# EUROPÄISCHE PATENTSCHRIFT

(11) **EP 3 639 782 B1**
(45) Veröffentlichungstag und Bekanntmachung des Hinweises auf die Patenterteilung: **24.12.2025**
(21) Anmeldenummer: 19202502.1
(22) Anmeldetag: 10.10.2019
(51) Int. Cl.: A61B 34/20

(54) **STEUERUNGSANORDNUNG ZUR STEUERUNG EINER BEWEGUNG EINES ROBOTERARMS**
CONTROL ARRANGEMENT FOR CONTROLLING A MOVEMENT OF A ROBOT ARM
DISPOSITIF DE COMMANDE D'UN MOUVEMENT D'UN BRAS ROBOTIQUE

(30) Priorität: 16.10.2018 DE 102018125592
(43) Veröffentlichungstag der Anmeldung: 22.04.2020
(73) Patentinhaber: Karl Storz SE & Co. KG, 78532 Tuttlingen (DE)
(72) Erfinder: Dr. Fallert, Johannes Albert, 78532 Tuttlingen (DE); Köhler, Benedikt, 78532 Tuttlingen (DE); Dr. Schrader, Stephan, 78532 Tuttlingen (DE); Dr. Zhang, Yaokun, 78532 Tuttlingen (DE)

(56) Entgegenhaltungen:
- WO-A1-2015/163943
- DE-A1- 102004 052 753
- DE-A1- 102008 041 260
- DE-A1- 102010 031 943
- DE-A1- 102012 110 190
- DE-A1- 102013 012 397
- DE-A1- 102016 107 853
- US-A1- 2013 063 580
- US-A1- 2018 071 029

## Beschreibung

Die Erfindung betrifft eine Steuerungsanordnung, ein Steuerungsverfahren zur Steuerung einer Bewegung eines Roboterarms, sowie eine Behandlungsvorrichtung mit der Steuerungsanordnung.

Aus dem Stand der Technik sind kombinierte chirurgische Halte- und Kamerasysteme bekannt, die für mikroskopische oder endoskopische Anwendungen auf verschiedene Weisen positioniert und gesteuert werden können. So können bspw. endoskopische Haltearme manuell oder über ein automatisches Positioniersystem, wie einen Roboterarm, bewegt und verstellt werden.

Die DE 10 2012 220 116 A1 beschreibt eine mobil handhabbare medizinische Vorrichtung, die einen Vorrichtungskopf, eine Führungseinrichtung zur Navigation, eine Kamera und eine Bilddaten verarbeitende Datenverarbeitungseinheit aufweist. Es ist eine Navigationsvorrichtung vorgesehen, mittels der der Vorrichtungskopf unter Verwendung einer vorbestimmten Karte an oder in einem Patienten navigiert werden kann.

Aus der DE 10 2007 054 450 A1 ist eine Vorrichtung zur Bereitstellung von Bildern für einen Operateur bekannt, wobei ein Roboterarm mit einer Mikroskop-Kamera vorgesehen ist. Mittels eines Navigationssystems wird sowohl das Operationsfeld als auch die Position der Kamera und deren Orientierung bestimmt.

Bei navigierten Instrumenten tritt das Problem auf, das diese Instrumente über ein optisches Tracking verfolgt werden müssen, wodurch das System sehr komplex wird. Es muss eine stete Sichtverbindung zwischen zu trackendem Instrument und Kamera bestehen; ferner ist elektromagnetisches Tracking störanfällig, je nachdem welche metallischen Verbindungen sich noch in dem Operationsbereich befinden.

Die DE 10 2014 188 962 A1 zeigt eine Anordnung zur Lagebestimmung eines chirurgischen Instruments, wobei das Instrument in einen Trokar geführt werden kann. Das Instrument weist ein Gyrometer und einen Beschleunigungssensor auf, wobei der Trokar eine Sensoranordnung zur Bestimmung einer Eindringtiefe aufweist.

Hierbei kann es notwendig werden, dass die Position des Instruments mittels des Inertialsensors bestimmt werden kann, jedoch kann der Sichtwinkel der Kamera nicht passen - der Arzt muss dann den Operationsvorgang unterbrechen, die Instrumente zur Seite legen und die Kamera richtig einstellen - dies kostet Zeit und unterbricht den Arbeitsfluss der Operation. Zudem kann der Arzt dazu verleitet werden aus Bequemlichkeit mit nicht optimalen Sichtwinkeln oder unscharfen Bildern zu arbeiten.

Ausgehend von diesem Stand der Technik ist es Aufgabe der vorliegenden Erfindung, eine Steuerungsanordnung zur Steuerung einer Bewegung eines Roboterarms vorzuschlagen, die eine exakte Positionierung des Roboterarms bietet, dabei eine Unterbrechung des Arbeitsflusses verhindert und einfach zu bedienen ist.

Die Patentanmeldung DE 10 2016 107 853 A1 offenbart einen Operations-Assistenz-Roboter zur Führung medizinischer Hilfsinstrumente, z. B. einer Endoskopkamera. Dazu weist ein medizinisches Instrument eine Sensoreinheit auf, die Beschleunigungs- und Rotationsdaten erfasst und mit einer Steuereinheit verbunden ist. Aufgrund dieser erfassten Daten lässt sich der Operations-Assistenz-Roboter durch das Instrument steuern.

Die Offenlegungsschrift DE 10 2010 031 943 A1 lehrt die Führung eines medizinischen Instruments mit Hilfe einer Orientierungsvorrichtung in Form eines Neigungsmessers. Dieser kann aus einer Wasserwaage, einem Beschleunigungsmesser oder einem MEMS bestehen.

Die Patentanmeldung DE 10 2004 052 753 A1 beschreibt ein Operations-Assistenz-System mit einer motorisierten Kinematik. Mittels dieser kann ein Hilfsinstrument während der Operation gehalten und bewegt werden. Um es einem führenden Instrument nachzuführen, werden dessen Positionskoordinaten von elektromagnetischen und /oder optischen Sensorelementen erfasst und die motorisierte Kinematik entsprechend angesteuert.

Die Patentanmeldung US 2018/0071029 A1 lehrt die Verwendung von Beschleunigungs-, Kraft-, Gyroskop-, Magnetsensoren sowie Dehnmessstreifen im Zusammenhang mit einem Verfolgungssystem im Bereich der Neurochirurgie.

Die Anmeldung DE 10 2008 041 260 A1 offenbart die Beschränkung der Bewegung auf einen definierten Arbeitsbereich.

Ausgehend von diesem Stand der Technik ist es Aufgabe der vorliegenden Erfindung, eine Steuerungsanordnung zur Steuerung einer Bewegung eines Roboterarms vorzuschlagen, die eine exakte Positionierung des Roboterarms bietet, dabei eine Unterbrechung des Arbeitsflusses verhindert und einfach zu bedienen ist.

Diese Aufgabe wird durch eine Steuerungsanordnung mit den Merkmalen des Anspruchs 1 gelöst.

Es ist ferner Aufgabe der Erfindung eine Behandlungsvorrichtung bereitzustellen, die eine sichere und effektive Behandlung eines Patienten ermöglicht, indem eine exakte Aufnahme des Arbeitsbereichs ermöglicht wird. Diese Aufgabe wird durch eine Behandlungsvorrichtung mit den Merkmalen des Anspruchs 8 gelöst.

Die weitere Aufgabe, ein Steuerungsverfahren zur Steuerung einer Bewegung eines Roboterarms vorzuschlagen, das eine exakte Positionierung des Roboterarms ermöglicht, ohne den Arbeitsfluss einer Operation zu unterbrechen, wird durch die Steuerungsanordnung des abhängigen Anspruchs 9 gelöst.

Bevorzugte Ausführungsformen der Steuerungsanordnung, der Behandlungsvorrichtung und des Steuerungsverfahrens sind in den Unteransprüchen ausgeführt.

Eine erste Ausführungsform der erfindungsgemäßen Steuerungsanordnung ist zur Steuerung einer Bewegung eines Roboterarms innerhalb eines vorgegebenen Arbeitsbereichs des Roboterarms ausgebildet. Dabei weist die Steuerungsanordnung zumindest auf: Ein chirurgisches Instrument mit einem Inertialsensor, einen Roboterarm mit einer an einem distalen Ende des Roboterarms angeordneten Kameraeinheit und eine Freigabe-Einheit, die mit dem Inertialsensor operativ gekoppelt ist und basierend auf den Inertialsensorsignalen eine Bewegung des chirurgischen Instruments erfasst, wobei in Abhängigkeit einer erfassten Bewegung des chirurgischen Instruments eine Steuerungsbewegung des Roboterarms, der mit einer Steuereinheit zur Steuerung gekoppelt ist, auslösbar oder beendbar ist.

Endoskopische Kameras, Exoskope oder chirurgische Instrumente können mit einer speziellen Sensorik ausgestattet werden - durch Auslesen dieser Sensoren zu bestimmten Zeitpunkten bzw. während einer durch den Anwender ausgeführten Bewegung können Befehle an den Haltearm oder eine Kameraeinheit weitergegeben werden und dadurch eine exakte Positionierung des Roboterarms erreicht werden.

In einer weiteren Ausführungsform der erfindungsgemäßen Steuerungsanordnung können das chirurgische Instrument, der Roboterarm, die Kameraeinheit und die Freigabe-Einheit über die Steuereinheit operativ miteinander gekoppelt sein. Unter "operativ gekoppelt" wird unter Bezug auf "Operation" die Kopplung eines Vorgangs oder eines Arbeitsvorgangs mit einem dadurch initiierten Vorgang oder Arbeitsvorgang verstanden, so dass die Steuereinheit und die übrigen elektronischen Komponenten miteinander drahtlos/drahtgebunden kommunizieren und Daten zur Auswertung, Speicherung und Steuerung austauschen.

"Anordnung" meint hierbei eine Verbindung der elektronischen Komponenten, so dass sie zusammenarbeiten, dabei Daten austauschen und eine Aktion einer Komponente zu einer Reaktion einer anderen Komponente innerhalb der Anordnung führt. Dabei liegen die Komponenten derart im Raum vor, dass sie räumlich benachbart zueinander sind und damit eine Arbeitseinheit, also eine Anordnung, wobei jede Komponente ihre spezifische Aufgabe hat, bilden können.

Ein "Roboterarm" im Sinne der Erfindung ist ein Industrieroboter oder Teil desselben und ist als Manipulator mit Werkzeugen und Aktoren ausgestattet. Solche Roboterarme sind universell einsetzbare Bewegungsautomaten mit mehreren Achsen, deren Bewegungen hinsichtlich Bewegungsfolge und Wegen bzw. Winkeln frei, ohne mechanischen bzw. menschlichen Eingriff, programmierbar und sensorgeführt sind. In der erfindungsgemäßen Anordnung kann der Roboterarm ein Werkzeug, eine Haltevorrichtung bzw. die Kameraeinheit oder eine Kombination daraus tragen. Der Roboterarm kann auch als eigentlicher Aktor verstanden werden, der ein Endoskop oder ein Exoskop, das an dem distalen Ende des Roboters befestigt ist, bewegt.

Eine "Steuerungsbewegung" beinhaltet jede Bewegung des Roboterarms, die mit der Umpositionierung des Arms, der Kameraeinheit, der Änderungen und Anpassung der Kamera-Freiheitsgrade in der Kameraeinheit sowie mit weiteren Bewegungen zur Verbesserung einer Sicht auf den Arbeitsbereich oder Bildqualität verbunden sind.

Bevorzugt ist der Inertialsensor sowie die Freigabe-Einheit mit der Steuereinheit des Roboterarms operativ gekoppelt, um ein Freigabe-Signal, sowie die Bewegung des Instruments, ergo eines Signals des Inertialsensors, verarbeiten zu können.

Eine weitere Ausführungsform der erfindungsgemäßen Steuerungsanordnung kann vorsehen, dass das chirurgische Instrument eine Handhabe aufweist, wobei der Inertialsensor in der Handhabe angeordnet ist. Damit ist die Bewegung der Hand eines Benutzers mit der Bewegung der Handhabe gekoppelt, wodurch die Bestimmung der Position und der Orientierung des chirurgischen Instruments im Arbeitsbereich exakter ist.

In einer Ausführungsform der erfindungsgemäßen Steuerungsanordnung kann der Inertialsensor ein MEMS-Sensor sein, der einen oder mehrere Sensor(en) ausgewählt aus der Gruppe Beschleunigungssensor, Gyroskop und Magnetometer aufweist. Vorteilhaft können 3D-Sensoren und kombinierte Mehrfachsensorvorrichtungen direkt in der Handhabe des chirurgischen Instruments integriert sein. Gerade im Bereich der Endoskopie, insbesondere der Neuro-Endoskopie, bietet es sich an, Inertialsensoren in bestimmte chirurgische Instrumente einzusetzen, die während der Operation in etwa 80 % der Operationszeit in der Hand gehalten werden. Als chirurgisches Instrument kann ein Sauger vorgesehen sein. Es kommen aber auch Endoskope, Fräsen oder beliebige andere Instrumente in Frage.

In noch einer weiteren Ausführungsform der Steuerungsanordnung kann der Roboterarm an seinem distalen Ende einen Aktor aufweisen, der mittels des Roboterarms bewegt werden kann. Der Aktor kann ein weiteres chirurgisches Instrument, bspw. ein Endoskop, ein medizinisches Hilfsmittel, wie ein Trokar oder einfach eine Haltevorrichtung sein.

Außerdem kann der Roboterarm dazu ausgebildet sein, eine Position und eine Orientierung der Kameraeinheit und einen oder mehrere Freiheitsgrad(e) der Kameraeinheit aus der Gruppe umfassend Fokus, Auswahl eines oder mehrerer Fokuspunkte, Auswahl eines Bildausschnitts, Verschwenken (Bewegung in x- /y- / z-Richtung), sowie Schwenken (Pivotieren, Bewegung in x- / y-Richtung um einen gewählten Fokuspunkt), Zoom und Änderung des Arbeitsabstandes zu steuern. Ferner ist eine Kippung, Translation in allen drei Raumrichtungen bzw. Rotation der Kameraeinheit mittels des Roboterarms möglich. Jede vorbeschriebene Bewegung bzw. Freiheitsgradänderung kann einzeln erfolgen oder in beliebiger Kombination. Damit kann der Roboterarm dazu verwendet werden, die Kameraeinheit nicht nur zu tragen, sondern auch verschiedene Einstellungen für die Kameraeinheit einzunehmen und sie damit zu "steuern". Das Gleiche kann für einen Aktor erfolgen, der an dem distalen Ende des Roboterarms angeordnet sein kann. Dazu und unabhängig davon kann auch eine distale Spitze des Roboterarms von der Kameraeinheit erfasst werden, um eine zusätzliche Möglichkeit zur Positionsbestimmung zu erhalten und in einem endoskopischen bzw. exoskopischen Bild weitere Freiheitsgrade anzusteuern.

Ferner liegt in einer weiteren Ausführungsform der erfindungsgemäßen Steuerungsanordnung die Freigabe-Einheit mechanisch entkoppelt von dem chirurgischen Instrument in der Steuerungsanordnung vor. Bevorzugt kann die Freigabe-Einheit ein Fußtaster sein. Alternativ sind Bedienknöpfe oder andere elektronische Auslöse-Einheiten möglich. Wichtig ist eine ergonomische Anordnung, so dass die Freigabe-Einheit als Bedienelement für einen Benutzer einfach zu erreichen und zu nutzen ist.

In noch einer weiteren Ausführungsform der erfindungsgemäßen Steuerungsanordnung ist der Arbeitsbereich für die Bewegung des Roboterarms derart dimensioniert, dass der Roboterarm innerhalb vorbestimmter Abmessungen bewegt werden kann. Unter dem Arbeitsbereich wird der räumlich abgesteckte und fest dimensionierte Bereich verstanden, innerhalb dessen der Roboterarm sich bewegen kann, ohne Personen, die sich in der Nähe des Arbeitsbereiches aufhalten können, zu verletzen oder andere Gegenstände zu berühren. Der Arbeitsbereich in der erfindungsgemäßen Steuerungsanordnung umfasst bevorzugt den Bereich, in dem das Instrument eingesetzt wird, also den Raum, in dem der Roboter oder Roboterarm und damit die mit dem Roboterarm verbundene Kameraeinheit nicht mit Patienten, OP-Personal sowie anderen Gegenständen in OP-Umgebung kollidieren können. Eine Bewegung des Roboterarms außerhalb des Arbeitsbereichs ist nicht zulässig. Der Arbeitsbereich kann statisch in präoperativer Planung vordefiniert sein, als auch durch intraoperative Überwachung durch geeignete Sensoren dynamisch (bevorzugt in Echtzeit) erfasst und angepasst werden.

In noch einer weiteren Ausführungsform der Steuerungsanordnung ist ein mit der Steuereinheit operativ gekoppelter Controller zur Übertragung der durch den Inertialsensor erfassten Signale, insbesondere der Orientierungs- bzw. Bewegungsfreiheitsgrade, des chirurgischen Instruments vorgesehen. Dieser Controller kann in der Steuereinheit vorliegen oder auch direkt mit dem chirurgischen Instrument verbunden sein. Mittels der Steuereinheit kann dann aufgrund der erfassten Werte eine Steuerung des Roboterarms initiiert werden. Die Steuerung kann dabei nach zwei Arten erfolgen: In einer "absoluten Steuerung" kann nach Freigabe die Steuerung rein positionsbezogen erfolgen, d. h. bspw. eine Winkeländerung, die durch die mit dem Inertialsensor erfasste Bewegung des chirurgischen Instruments initiiert wird, wird in eine Endposition des Roboterarms bzw. einen bestimmten internen Freiheitsgrad der Kameraeinheit geändert. Erfindungsgemäss wird in einer "Geschwindigkeits-Steuerung" die angesprochene Winkeländerung in eine Geschwindigkeitsänderung umgewandelt, wonach die Geschwindigkeit der Bewegung des Roboterarms bzw. die Änderungsgeschwindigkeit der internen Freiheitsgrade der Kameraeinheit angepasst wird. In noch einer weiteren Möglichkeit kann eine relative Steuerung vorgesehen sein, wobei Neigung, Rotation oder Lage des Sensors in Bezug auf ein vorbestimmtes Weltkoordinatensystem zu einer kontinuierlichen Bewegung des Roboterarms (ohne Endposition) führt, die umso schneller ist, je weiter die Auslenkung des Sensors relativ zum Koordinatensystem ist. Ein Anhalten der Bewegung erfolgt entweder, wenn das Instrument keinen relativen Abstand (Delta) zum Weltkoordinatensystem aufweist oder durch die Freigabeeinheit unterbrochen wird.

Eine erfindungsgemäße Behandlungsvorrichtung mit einem Behandlungstisch für einen Patienten weist einen Roboterarm auf, der zur Durchführung einer Behandlung dem Behandlungstisch für einen Patienten zugeordnet ist. Ferner weist die Behandlungsvorrichtung eine erfindungsgemäße Steuerungsanordnung zur Steuerung einer Bewegung des Roboterarms auf. Vorteilhaft kann eine Operation mit der erfindungsgemäßen Steuerungsanordnung erfolgen, deren Arbeitsfluss nicht unterbrochen werden muss. Durch die Freigabe-Einheit kann ein operierender Arzt sehr einfach eine Bewegung des Roboterarms oder Anpassung der Kameraeinheit vornehmen, ohne die Operation unterbrechen oder anderweitig verzögern zu müssen. Klare Bilder und eine gute Sicht werden ermöglicht.

Um die aufgenommenen Bilder darzustellen, kann die Steuerungsanordnung einen Monitor aufweisen, der mit der Kamerakontrolleinheit und der Steuereinheit des Roboterarms verbunden ist. Darauf können kontinuierlich das aktuelle Kamerabild sei es von dem Arbeitsbereich des Roboterarms als auch eines Endo- oder Exoskops sein, das mit dem Roboterarm verbunden ist.

Es kann vorgesehen sein, dass in der Behandlungsvorrichtung der Arbeitsbereich für die Bewegung des Roboterarms definiert ist, wobei der Arbeitsbereich derart dimensioniert ist, dass der Roboterarm innerhalb vorbestimmter Abmessungen, die den Behandlungstisch einschließen, bewegt werden kann. Die Abmessungen geben einen vorgegebenen Arbeitsbereich vor und sind der jeweiligen Behandlungsvorrichtung, ob der Patient waagrecht liegt oder erhöht liegt oder sitzt, angepasst werden. Der Roboterarm hält bei Bewegung in Richtung Rand des Arbeitsbereiches in seiner Position automatisch am Rand des Arbeitsbereiches inne. Die Steuerungen, die den Roboter weiter in die Richtung außerhalb des Arbeitsbereiches steuert, werden automatisch von der Steuerungseinheit unterdrückt. Nur die Steuerung in Rückwärtsrichtung wird durchgeführt.

Ein erfindungsgemäßes Steuerungsverfahren zur Steuerung einer Bewegung eines Roboterarms in einer erfindungsgemäßen Steuerungsanordnung umfasst in einer ersten Ausführungsform die folgenden Schritte:
a) Aktivieren eines Bewegungsmodus des Roboterarms durch Bedienen der Freigabe-Einheit, dabei gleichzeitig
   a1) Erfassen eines Werts des Inertialsensors und Bestimmen einer Referenzorientierung und / oder einer Referenzposition des chirurgischen Instruments innerhalb des vorgegebenen Arbeitsbereichs, und
   a2) Erfassen eines Bildes des Arbeitsbereichs mit der Kameraeinheit und daraus Bestimmen einer Referenzorientierung und / oder einer Referenzposition des chirurgischen Instruments in dem Arbeitsbereich, dann
b) Bewegen des chirurgischen Instruments, dabei gleichzeitig
   b1) kontinuierlich Erfassen von Werten des Inertialsensors und Weitergeben der erfassten Werte an die Steuereinheit, und
   b2) mit der Kameraeinheit kontinuierlich Erfassen von Bildern des Arbeitsbereichs und daraus Bestimmen einer Relativorientierung und / oder Relativposition des chirurgischen Instruments in dem Arbeitsbereich in Bezug zu den zuvor erfassten Referenzorientierung und / oder Referenzposition; und
c) aus der vorbestimmten Referenzorientierung und / oder der vorbestimmten Referenzposition des chirurgischen Instruments und der Relativorientierung und / oder Relativposition des chirurgischen Instruments, die aus der Bewegung des chirurgischen Instruments und der gleichzeitigen Erfassung des Arbeitsbereichs durch die Kameraeinheit bestimmt wurden, Bestimmen einer Bewegungsbahn des chirurgischen Instruments und funktionsabhängig Ansteuern des Roboterarms, dabei Bewegen des Roboterarms innerhalb des Arbeitsbereichs und / oder Anpassen von Kamerafreiheitsgraden der Kameraeinheit,
d) Deaktivieren des Bewegungsmodus des Roboterarms durch erneutes Bedienen der Freigabe-Einheit.

Vorteilhaft kann ein Arzt während einer Operation eine intuitive Steuerungsmethode nutzen. Mit dem erfindungsgemäßen Verfahren wird eine Umpositionierung, Steuerung der Kamerafreiheitsgrade bzw. -parameter und direkte Positionierung im Arbeitsbereich ermöglicht, ohne dass der Arzt seine Instrumente aus dem Situs nehmen muss oder seine gewohnte Arbeitshaltung verändern muss - ein durchgängiger Arbeitsablauf der Operation wird dadurch ermöglicht.

Zum funktionsabhängig Ansteuern des Roboterarms kann folgendes vorgesehen sein: Bei Aktivieren wird eine erste Orientierung des chirurgischen Instruments und der Kameraeinheit und Bestimmen dieser Orientierung als Ausgangsorientierung. Hiernach wird eine Bewegungsrichtung des chirurgischen Instruments und eine Kamerabildrichtung der Kameraeinheit bestimmt, indem vorbestimmte Orientierungspunkte in dem Arbeitsbereich des Roboterarms erfasst werden. Daraus können eine relative Orientierung in Bezug zu der Ausgangsorientierung und ein Neigungswinkel des chirurgischen Instruments relativ zu der Ausgangsorientierung bestimmt werden. Die Kameraeinheit wird in derselben Richtung bewegt wie der Neigungswinkel des chirurgischen Instruments zeigt, wobei eine Bewegung bis zum Deaktivieren oder bis zu der Grenze des Arbeitsbereichs durchgeführt wird. Dabei wird eine Bewegungsgeschwindigkeit der Kameraeinheit proportional zu einem Wert des Neigungswinkels oder zu einem Verhältnis aus Ausgangsorientierung und relativer Orientierung gewählt.

Anders gesprochen, heißt das, jedes Mal, wenn die Steuerung aktiviert wird (z. B. über die Betätigung eines Fußpedals oder eines Aktivierungsknopfes, wird die aktuelle Orientierung des Instruments (Inertialsensor) als neutrale Ausgangs- oder Referenzorientierung oder auch -position festgestellt. Die Neigung bzw. der Neigungswinkel des Instruments relativ zum der Ausgangsorientierung wird als Eingabegröße der Steuerung interpretiert, so dass das chirurgische Instrument ähnlich wie ein Joystick verwendet werden kann. Eine Bewegung des Instruments in eine eindeutig definierte Ausrichtung des Instruments (erkennbar durch ein oder mehrere Orientierungspunkte als feste Merkmale auf dem chirurgischen Instrument, wie z. B. ein Loch auf einem Neurosauger), wird als Bewegung festgelegt, die bspw. ein "Bild nach oben"-Steuerungsbefehl definiert. Damit lässt sich automatisch die Bewegungen für das "Bild nach unten" (entgegengesetzt) sowie, Bild links und rechts" (jeweils senkrecht dazu) ableiten. Die Bewegungsrichtung des chirurgischen Instruments wird damit mit der Bildrichtung registriert, die mit der Kameraeinheit erfasst wird. Die Steuerung der Kameraeinheit kann auf zwei Weisen erfolgen: In einer ersten Ausführungsform kann der Roboterarm unendlich in die Richtung der Steuerungseingabe relativ zu seiner aktuellen Position bewegt werden, bis das chirurgische Instrument wieder zurück zur Ausgangsorientierung kommt oder die Steuerung deaktiviert wird, z. B. durch Loslassen des Fußpedals. Die Bewegungsgeschwindigkeit der Kameraeinheit ist dabei proportional zum Neigungswinkel. Damit ist die Steuerung einer beliebig großen Bewegung möglich, ohne das Instrument großartig zu bewegen. Eine leichte Neigung kann hierfür schon ausreichend sein. Eine zweite Möglichkeit ist, die aktuelle Orientierung der Kameraeinheit zum Zeitpunkt der Aktivierung der Steuerung auch als Ausgangsorientierung festzulegen. Der jeweilige Neigungswinkel des chirurgischen Instruments wird zu einem vorgegebenen Verhältnis zur Kameraeinheit übertragen, z. B. 1:5. Wenn das chirurgische Instrument dann ein Grad in Richtung "Bild rechts" relativ zu seiner Ausgangsorientierung oder -position gekippt wird, kippt die Kamera in Bildrechts relativ zu seiner Ausgangsorientierung um fünf Grad, d. h. die Kameraeinheit führt die Bewegungen des chirurgischen Instruments skaliert nach.

Durch Mapping der ausgeführten Bewegungsmuster auf eine bestimmte Funktion kann eine intuitive Steuerung innerhalb der Steuerungsanordnung erreicht werden, ohne den Arbeitsfluss während eines medizinischen Eingriffs zu beeinflussen und eine sichere Operation zu ermöglichen. Weitere Ausführungsformen der Steuerungsanordnung und der Behandlungsvorrichtung sowie einige der Vorteile, die mit diesen und weiteren Ausführungsformen verbunden sind, werden durch die nachfolgende ausführliche Beschreibung unter Bezug auf die begleitende Figur deutlich und besser verständlich. Die Figur ist lediglich eine schematische Darstellung einer Ausführungsform der Erfindung.

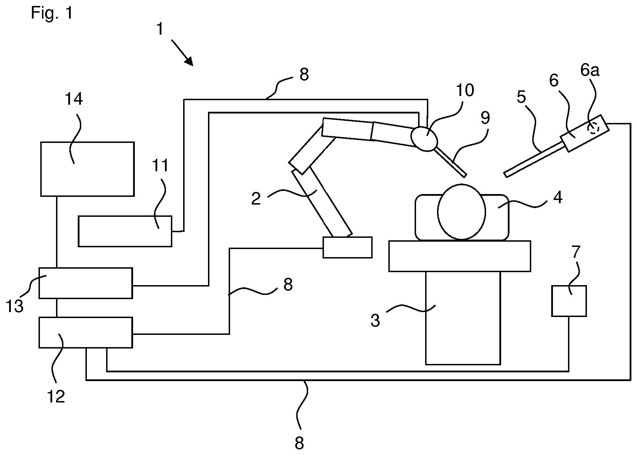

Dabei zeigt Fig. 1 eine schematische Ansicht der erfindungsgemäßen Behandlungsvorrichtung,

In Fig. 1 ist eine Behandlungsvorrichtung 1 dargestellt, die neben einem Roboterarm 2 einen Behandlungstisch 3 aufweist. Beispielhaft ist ein Patient 4 schematisch auf dem Behandlungstisch 3 liegend dargestellt; die Behandlungsvorrichtung 1 kann zu Übungszwecken oder anderen Zwecken aber auch ohne Patient 4 funktionieren.

Es ist zur Durchführung einer Operation ein chirurgisches Instrument 5 vorgesehen, das eine Handhabe 6 zum Halten und Führen des Instruments 5 aufweist. In der Handhabe 6 ist ein Inertialsensor 6a angeordnet, der zum Erfassen einer Orientierung bzw. Position der Handhabe 6 und damit des chirurgischen Instruments 5 dient.

Der Roboterarm 2 weist neben beweglichen Gliedern einen Roboterkopf 10 auf, an dem eine Kameraeinheit 9 montiert ist. Der Roboterkopf 10, also das distale Ende des Roboterarms 2, ist elektronisch über elektrische Leitungen 8 mit einer Kamerakontrolleinheit 13 sowie einem Monitor 14 und der Steuereinheit 12 verbunden.

Mechanisch unabhängig von der Handhabe 6 ist eine Freigabe-Einheit 7 angeordnet, die in Fig.1 als Fußtaster 7 dargestellt ist. Mit der Freigabe-Einheit 7 wird ein Bewegungsmodus des Roboterarms 2 ausgelöst bzw. wieder beendet. Während des Bewegungsmodus wird die Bewegung der Handhabe 6 durch kontinuierliches Auslesen des Inertialsensors 6a erfasst und mit einer von der Kameraeinheit 9 erfassten Bewegung der Handhabe 6 konsolidiert - daraufhin kann die Kameraeinheit 9 neu positioniert werden oder Einstellungen, wie Fokus oder Winkel eingestellt werden.

Die vorliegende Erfindung stellt eine Behandlungsvorrichtung bereit. Die erfindungsgemäße Steuerungsanordnung ist zur Steuerung einer Bewegung eines Roboterarms 2 innerhalb eines vorgegebenen Arbeitsbereichs des Roboterarms 2 ausgebildet. Erfindungsgemäß weist die Steuerungsanordnung 1 zumindest auf: die Merkmale des Anspruchs 1.

### Bezugszeichenliste

- 1: Anordnung
- 2: Roboterarm
- 3: Behandlungstisch
- 4: Patient
- 5: Instrument
- 6: Handhabe
- 6a: Inertialsensor
- 7: Fußtaster/Freigabe-Einheit
- 8: Leitung
- 9: Kameraeinheit
- 10: Roboterkopf
- 11: Lichtquelle
- 12: Roboter/Aktor-Steuereinheit
- 13: Kamerakontrolleinheit
- 14: Monitor

## Patentansprüche

1. Steuerungsanordnung, die zur Steuerung einer Bewegung eines Roboterarms (2) innerhalb eines vorgegebenen Arbeitsbereichs des Roboterarms (2) ausgebildet ist,
wobei die Steuerungsanordnung (1) zumindest aufweist:
- ein chirurgisches Instrument (5) mit einem Inertialsensor (6a),
- einen Roboterarm (2) mit einer an einem distalen Ende des Roboterarms (2) angeordneten Kameraeinheit (9),
- eine Freigabe-Einheit (7), die mit dem Inertialsensor (6a) operativ gekoppelt ist und basierend auf den Inertialsensorsignalen eine Bewegung des chirurgischen Instruments (5) erfasst, wobei in Abhängigkeit einer erfassten Bewegung des chirurgischen Instruments (5) eine Steuerungsbewegung des Roboterarms (2), der mit einer Steuereinheit (12) zur Steuerung gekoppelt ist, auslösbar oder beendbar ist,
wobei eine Bewegung des Roboterarms (2) außerhalb des Arbeitsbereichs unterdrückbar ist, wobei
die Steuerungsanordnung (1) dazu eingerichtet ist, die Kameraeinheit (9) zu bewegen in eine Bildrichtung, die einer eindeutig definierten Ausrichtung des Instruments (5) entspricht, und die in die selbe Richtung wie ein Neigungswinkel des chirurgischen Instruments (5) zeigt, wobei die Bewegung bis zum Deaktivieren oder bis zu der Grenze des Arbeitsbereichs durchgeführt wird, wobei eine Bewegungsgeschwindigkeit der Kameraeinheit proportional zu einem Wert des Neigungswinkels oder zu einem Verhältnis aus Ausgangsorientierung und relativer Orientierung gewählt ist.

2. Steuerungsanordnung nach Anspruch 1,
**dadurch gekennzeichnet, dass**
das chirurgische Instrument (5) eine Handhabe (6) aufweist, wobei der Inertialsensor (6a) in der Handhabe (6) angeordnet ist.

3. Steuerungsanordnung nach Anspruch 1 oder 2,
**dadurch gekennzeichnet, dass**
der Inertialsensor (6a) ein MEMS-Sensor ist, der zumindest einen Sensor ausgewählt aus der Gruppe Beschleunigungssensor, Gyroskop und Magnetometer aufweist.

4. Steuerungsanordnung nach zumindest einem der Ansprüche 1 bis 3,
**dadurch gekennzeichnet, dass**
der Roboterarm (2) an seinem distalen Ende einen Aktor aufweist, der mittels des Roboterarms (2) bewegbar ist.

5. Steuerungsanordnung nach Anspruch 4,
**dadurch gekennzeichnet, dass**
der Roboterarm (2) dazu ausgebildet ist, eine Position und eine Orientierung der Kameraeinheit (9) und zumindest einen Freiheitsgrad der Kameraeinheit (9) aus der Gruppe umfassend Fokus, Auswahl eines Bildausschnitts, Verschwenken und Zoom, zu steuern.

6. Steuerungsanordnung (1) nach zumindest einem der Ansprüche 1 bis 5,
**dadurch gekennzeichnet, dass**
die Freigabe-Einheit (7) mechanisch entkoppelt von dem chirurgischen Instrument (5) in der Anordnung (1) vorliegt, wobei die Freigabe-Einheit bevorzugt ein Fußtaster (7) ist.

7. Steuerungsanordnung (1) nach zumindest einem der Ansprüche 1 bis 6,
**dadurch gekennzeichnet, dass**
der Arbeitsbereich für die Bewegung des Roboterarms (2) derart dimensioniert ist, dass der Roboterarm (2) innerhalb vorbestimmter Abmessungen bewegbar ist.

8. Behandlungsvorrichtung mit einem Behandlungstisch (3) für einen Patienten (4), wobei die Behandlungsvorrichtung
- einen Roboterarm (2), der zur Durchführung einer Behandlung dem Behandlungstisch (3) für einen Patienten (4) zugeordnet ist,
- und eine Steuerungsanordnung zur Steuerung einer Bewegung eines Roboterarms (2), aufweist
**dadurch gekennzeichnet, dass**
die Steuerungsanordnung zur Steuerung einer Bewegung eines Roboterarms (2) eine Steuerungsanordnung (1) nach zumindest einem der Ansprüche 1 bis 7 ist.

9. Steuerungsanordnung (1), nach zumindest einem der Ansprüche 1 bis 7, die dazu eingerichtet ist, ein Steuerungsverfahren auszuführen, das Steuerungsverfahren **umfassend die Schritte**
a) Aktivieren eines Bewegungsmodus des Roboterarms (2) durch Bedienen der Freigabe-Einheit (7), dabei gleichzeitig
a1) Erfassen eines Werts des Inertialsensors (6a) und Bestimmen einer Referenzorientierung und / oder einer Referenzposition des chirurgischen Instruments (5) innerhalb des vorgegebenen Arbeitsbereichs, und
a2) Erfassen eines Bildes des Arbeitsbereichs mit der Kameraeinheit (9) und daraus Bestimmen einer Referenzorientierung und / oder einer Referenzposition des chirurgischen Instruments (5) in dem Arbeitsbereich, dann
b) Bewegen des chirurgischen Instruments (5), dabei gleichzeitig
b1) kontinuierlich Erfassen von Werten des Inertialsensors und Weitergeben der erfassten Werte an die Steuereinheit (12), und
b2) mit der Kameraeinheit (9) kontinuierlich Erfassen von Bildern des Arbeitsbereichs und daraus Bestimmen einer Relativorientierung und / oder Relativposition des chirurgischen Instruments (5) in dem Arbeitsbereich in Bezug zu den zuvor erfassten Referenzorientierung und / oder Referenzposition; und
c) aus der vorbestimmten Referenzorientierung und / oder der vorbestimmten Referenzposition des chirurgischen Instruments (5) und der Relativorientierung und / oder Relativposition des chirurgischen Instruments (5), die aus der Bewegung des chirurgischen Instruments (5) und der gleichzeitigen Erfassung des Arbeitsbereichs durch die Kameraeinheit (9) bestimmt wurden, Bestimmen einer Bewegungsbahn des chirurgischen Instruments (5) und funktionsabhängig Ansteuern des Roboterarms (2), dabei Bewegen des Roboterarms (2) innerhalb des Arbeitsbereichs und / oder Anpassen von Kamerafreiheitsgraden der Kameraeinheit (9),
d) Deaktivieren des Bewegungsmodus des Roboterarms (2) durch erneutes Bedienen der Freigabe-Einheit (7).

10. Steuerungsanordnung (1) nach Anspruch 9, das Steuerungsverfahren **umfassend den Schritt**
zum funktionsabhängig Ansteuern des Roboterarms (2)
- bei Aktivieren Erfassen einer ersten Orientierung des chirurgischen Instruments (5) und der Kameraeinheit (9) und Bestimmen dieser Orientierung als Ausgangsorientierung;
- Bestimmen einer Bewegungsrichtung des chirurgischen Instruments (5) und einer Kamerabildrichtung der Kameraeinheit (9) durch Erfassen vorbestimmter Orientierungspunkte in dem Arbeitsbereich des Roboterarms (2);
- daraus Bestimmen einer relativen Orientierung in Bezug zu der Ausgangsorientierung und Bestimmen eines Neigungswinkel des chirurgischen Instruments (5) relativ zu der Ausgangsorientierung;
- Bewegen der Kameraeinheit (9) in der selben Richtung wie der Neigungswinkel des chirurgischen Instruments (5) zeigt, wobei eine Bewegung bis zum Deaktivieren oder bis zu der Grenze des Arbeitsbereichs durchgeführt wird,
wobei eine Bewegungsgeschwindigkeit der Kameraeinheit (9) proportional zu einem Wert des Neigungswinkels oder zu einem Verhältnis aus Ausgangsorientierung und relativer Orientierung gewählt ist.

## Claims

1. A control arrangement which is designed to control a movement of a robot arm (2) within a predefined working region of the robot arm (2),
wherein the control arrangement (1) at least has:
- a surgical instrument (5) with an inertial sensor (6a),
- a robot arm (2) with a camera unit (9) arranged at a distal end of the robot arm (2),
- a release unit (7) which is operatively coupled to the inertial sensor (6a) and records a movement of the surgical instrument (5) based on the inertial sensor signals, wherein a control movement of the robot arm (2), which is coupled to a control unit (12) for control, can be triggered or terminated as a function of a recorded movement of the surgical instrument (5), wherein a movement of the robot arm (2) outside the working region can be suppressed, wherein the control arrangement (1) is configured to move the camera unit (9) in an image direction which corresponds to a clearly defined alignment of the instrument (5) and which points in the same direction as an angle of inclination of the surgical instrument (5), wherein the movement is carried out up to deactivation or up to the limit of the working region, wherein a movement speed of the camera unit is selected in proportion to a value of the angle of inclination or to a ratio of initial orientation and relative orientation.

2. The control arrangement according to claim 1,
**characterised in that**
the surgical instrument (5) has a handle (6), wherein the inertial sensor (6a) is arranged in the handle (6).

3. The control arrangement according to claim 1 or 2,
**characterised in that**
the inertial sensor (6a) is a MEMS sensor having at least one sensor selected from the group of an acceleration sensor, gyroscope and magnetometer.

4. The control arrangement according to at least one of claims 1 to 3,
**characterised in that**
the robot arm (2) has, at its distal end, an actuator, which can be moved by means of the robot arm (2).

5. The control arrangement according to claim 4,
**characterised in that**
the robot arm (2) is designed to control a position and an orientation of the camera unit (9) and at least one degree of freedom of the camera unit (9) from the group comprising focus, selection of an image section, panning and zoom.

6. The control arrangement (1) according to at least one of claims 1 to 5,
**characterised in that**
the release unit (7) is mechanically decoupled from the surgical instrument (5) in the arrangement (1), wherein the release unit is preferably a foot switch (7).

7. The control arrangement (1) according to at least one of claims 1 to 6,
**characterised in that**
the working region for the movement of the robot arm (2) is dimensioned in such manner that the robot arm (2) can be moved within predetermined dimensions.

8. A treatment device with a treatment table (3) for a patient (4), wherein the treatment device has
- a robot arm (2), which is assigned to the treatment table (3) for a patient (4) in order to carry out a treatment,
- and a control arrangement for controlling a movement of a robot arm (2),
**characterised in that**
the control arrangement for controlling a movement of a robot arm (2) is a control arrangement (1) according to at least one of claims 1 to 7.

9. The control arrangement (1)
according to at least one of claims 1 to 7, which is configured to carry out a control method, the control method
**comprising the steps**
a) Activating a movement mode of the robot arm (2) by operating the release unit (7), at the same time
a1) Recording a value of the inertial sensor (6a) and determining a reference orientation and/or a reference position of the surgical instrument (5) within the predefined working region, and
a2) Recording an image of the working region with the camera unit (9) and determining therefrom a reference orientation and/or a reference position of the surgical instrument (5) in the working region, then
b) Moving the surgical instrument (5) while at the same time
b1) Continuously recording values of the inertial sensor and relaying the recorded values to the control unit (12), and
b2) Continuously recording images of the working region with the camera unit (9) and determining therefrom a relative orientation and/or relative position of the surgical instrument (5) in the working region in relation to the previously recorded reference orientation and/or reference position; and
c) From the predetermined reference orientation and/or the predetermined reference position of the surgical instrument (5) and the relative orientation and/or relative position of the surgical instrument (5), which were determined from the movement of the surgical instrument (5) and the simultaneous recording of the working region by the camera unit (9), Determining a movement path of the surgical instrument (5) and activating the robot arm (2) depending on the function, thereby moving the robot arm (2) within the working region and/or adjusting camera degrees of freedom of the camera unit (9),
d) Deactivating the movement mode of the robot arm (2) by operating the release unit (7) again.

10. The control arrangement (1) according to claim 9, the control method **comprising the step**
for activating the robot arm (2) depending on the function
- When activated, recording a first orientation of the surgical instrument (5) and the camera unit (9) and determining this orientation as the initial orientation;
- Determining a movement direction of the surgical instrument (5) and a camera image direction of the camera unit (9) by recording predetermined orientation points in the working region of the robot arm (2);
- Determining therefrom a relative orientation in relation to the initial orientation and determining an angle of inclination of the surgical instrument (5) relative to the initial orientation;
- Moving the camera unit (9) in the same direction as the angle of inclination of the surgical instrument (5) points, wherein a movement is carried out up to the deactivation or up to the limit of the working region,
wherein a movement speed of the camera unit (9) is selected to be proportional to a value of the angle of inclination or to a ratio of the initial orientation and the relative orientation.

## Revendications

1. Agencement de commande, qui est conçu pour commander un déplacement d'un bras de robot (2) à l'intérieur d'une zone de travail spécifiée du bras de robot (2),
dans lequel l'agencement de commande (1) présente au moins :
- un instrument chirurgical (5) comportant un capteur inertiel (6a),
- un bras de robot (2) comportant une unité de caméra (9) agencée au niveau d'une extrémité distale du bras de robot (2),
- une unité de libération (7) qui est accouplée de manière opérationnelle au capteur inertiel (6a) et qui détecte un déplacement de l'instrument chirurgical (5) sur la base des signaux de capteur inertiel, dans lequel un déplacement de commande du bras de robot (2), qui est accouplé à une unité de commande (12) pour la commande, peut être déclenché ou arrêté en fonction d'un déplacement détecté de l'instrument chirurgical (5), dans lequel un déplacement du bras de robot (2) en dehors de la zone de travail peut être supprimé, dans lequel l'agencement de commande (1) est conçu pour déplacer l'unité de caméra (9) dans une direction d'image qui correspond à une orientation clairement définie de l'instrument (5) et qui pointe dans la même direction qu'un angle d'inclinaison de l'instrument chirurgical (5), dans lequel le déplacement est réalisé jusqu'à une désactivation ou jusqu'à la limite de la zone de travail, dans lequel une vitesse de déplacement de l'unité de caméra est choisie proportionnellement à une valeur de l'angle d'inclinaison ou à un rapport entre l'orientation initiale et l'orientation relative.

2. Agencement de commande selon la revendication 1,
**caractérisé en ce que**
l'instrument chirurgical (5) présente une poignée (6), dans lequel le capteur inertiel (6a) est agencé dans la poignée (6).

3. Agencement de commande selon la revendication 1 ou 2,
**caractérisé en ce que**
le capteur inertiel (6a) est un capteur MEMS, qui présente au moins un capteur choisi dans le groupe constitué par un accéléromètre, un gyroscope et un magnétomètre.

4. Agencement de commande selon au moins l'une des revendications 1 à 3,
**caractérisé en ce que**
le bras de robot (2) présente au niveau de son extrémité distale un actionneur, qui peut être déplacé par le biais du bras de robot (2).

5. Agencement de commande selon la revendication 4,
**caractérisé en ce que**
le bras de robot (2) est conçu pour commander une position et une orientation de l'unité de caméra (9) et au moins un degré de liberté de l'unité de caméra (9) parmi le groupe comprenant la mise au point, la sélection d'une partie d'image, le pivotement et le zoom.

6. Agencement de commande (1) selon au moins l'une des revendications 1 à 5,
**caractérisé en ce que**
l'unité de libération (7) est présente mécaniquement découplée de l'instrument chirurgical (5) dans l'agencement (1), dans lequel l'unité de libération est de préférence une pédale (7).

7. Agencement de commande (1) selon au moins l'une des revendications 1 à 6,
**caractérisé en ce que**
la zone de travail pour le déplacement du bras de robot (2) est dimensionnée de telle sorte que le bras de robot (2) peut être déplacé dans des dimensions prédéterminées.

8. Dispositif de traitement comportant une table de traitement (3) pour un patient (4), dans lequel le dispositif de traitement présente
- un bras de robot (2) qui est associé à la table de traitement (3) pour un patient (4) afin de réaliser un traitement,
- et un agencement de commande pour commander un déplacement d'un bras de robot (2),
**caractérisé en ce que**
l'agencement de commande pour commander un déplacement d'un bras de robot (2) est un agencement de commande (1) selon au moins l'une des revendications 1 à 7.

9. Agencement de commande (1),
selon au moins l'une des revendications 1 à 7, qui est conçu pour exécuter un procédé de commande, le procédé de commande
**comprenant les étapes**
a) d'activation d'un mode de déplacement du bras de robot (2) par manipulation de 'unité de libération (7), à cet effet simultanément
a1) de détection d'une valeur du capteur inertiel (6a) et de détermination d'une orientation de référence et/ou d'une position de référence de l'instrument chirurgical (5) à l'intérieur de la zone de travail spécifiée, et
a2) de détection d'une image de la zone de travail avec l'unité de caméra (9) et de détermination à partir de là d'une orientation de référence et/ou d'une position de référence de l'instrument chirurgical (5) dans la zone de travail, puis
b) de déplacement de l'instrument chirurgical (5), à cet effet simultanément
b1) de détection en continu de valeurs du capteur inertiel et de transmission des valeurs détectées à l'unité de commande (12), et
b2) avec l'unité de caméra (9), de détection en continu d'images de la zone de travail et de détermination à partir de là d'une orientation relative et/ou d'une position relative de l'instrument chirurgical (5) dans la zone de travail par rapport à l'orientation de référence et/ou la position de référence précédemment détectées ; et
c) à partir de l'orientation de référence prédéterminée et/ou de la position de référence prédéterminée de l'instrument chirurgical (5) et de l'orientation relative et/ou de la position relative de l'instrument chirurgical (5), qui ont été déterminées à partir du déplacement de l'instrument chirurgical (5) et de la détection simultanée de la zone de travail par l'unité de caméra (9), de détermination d'une trajectoire de déplacement de l'instrument chirurgical (5) et, en fonction de la fonction, de commande du bras de robot (2), du déplacement à cet effet du bras de robot (2) à l'intérieur de la zone de travail et/ou d'adaptation de degrés de liberté de caméra de l'unité de caméra (9),
d) de désactivation du mode de déplacement du bras du robot (2) par manipulation à nouveau de l'unité de libération (7).

10. Agencement de commande (1) selon la revendication 9, le procédé de commande
**comprenant l'étape**
pour la commande du bras du robot (2) en fonction de la fonction
- lors de l'activation, de détection d'une première orientation de l'instrument chirurgical (5) et de l'unité de caméra (9) et de détermination de cette orientation comme orientation initiale ;
- de détermination d'une direction de déplacement de l'instrument chirurgical (5) et d'une direction d'image de caméra de l'unité de caméra (9) par détection de points d'orientation prédéterminés dans la zone de travail du bras de robot (2) ;
- de détermination d'une orientation relative par rapport à l'orientation initiale et de détermination d'un angle d'inclinaison de l'instrument chirurgical (5) par rapport à l'orientation initiale ;
- de déplacement de l'unité de caméra (9) dans la même direction que celle dans laquelle pointe l'angle d'inclinaison de l'instrument chirurgical (5), dans lequel un déplacement est réalisé jusqu'à une désactivation ou jusqu'à la limite de la zone de travail,
dans lequel une vitesse de déplacement de l'unité de caméra (9) est choisie proportionnellement à une valeur de l'angle d'inclinaison ou à un rapport entre l'orientation initiale et l'orientation relative.
